# EUROPÄISCHE PATENTSCHRIFT

(11) **EP 0 136 465 B1**
(45) Veröffentlichungstag und Bekanntmachung des Hinweises auf die Patenterteilung: **17.05.1995**
(21) Anmeldenummer: 84109134.1
(22) Anmeldetag: 01.08.1984
(51) Int. Cl.: C07J 53/00

(54) **Verfahren zur Herstellung von 17alpha-Acetoxy-1alpha,2alpha-methylen-4,6-pregnadien-3,20-dion**
Process for the preparation of 17-alpha-acetoxy-1-alpha, 2-alpha-methylene-pregna-4,6-diene-3,20 dione
Procédé de préparation de 17 alpha-acétoxy-1 alpha, 2 alpha-methylène-pregna-4,6-diène-3,20-dione

(30) Priorität: 07.09.1983 DE 3332590
(43) Veröffentlichungstag der Anmeldung: 10.04.1985
(73) Patentinhaber: SCHERING AKTIENGESELLSCHAFT, 13342 Berlin (DE)
(72) Erfinder: Arnoldt, Hanfried, Dr., D-1000 Berlin 33 (DE)

(56) Entgegenhaltungen:
- DE-B- 1 096 353
- DE-B- 1 183 500
- US-A- 3 365 446
- Chem.Berichte 93 (1960) 1710
- Methodicum Chimicum Bd.5, 513-516; Thieme Verl. 1975
- J.FRIED und J.A.EDWARDS Organic Reactions in Steroid Chemistry, VolII, 100-126 Van Nostrand Reinhold Comp. 1972
- WIECHERT "Synthesen antiandrogener Steroide" in RASPE (Herausg.) Advances in the Biosciences, Berlin 1967
- DJERASSI (Herausg.) Steroid Reactions, 371-379, San Francisco, 1963

## Beschreibung

Die Erfindung betrifft ein Verfahren zur Herstellung von 17α-Acetoxy-1α.2α-methylen-4.6-pregnadien-3.20-dion aus 17α-Hydroxy-1.4.6-pregnatrien-3.20-dion, welches dadurch gekennzeichnet ist, daß man 17α-Hydroxy-1.4.6-pregnatrien-3.20-dion an der 20-Ketogruppe mit Ethylenglycol in Gegenwart eines Ameisensäuretrialkylesters und einer starken Säure ketalisiert, anschließend das 20-Ketalsteroid in an sich bekannter Weise in 1.2-Stellung mit Trimethylsulfoxoniumjodid in einem polaren aprotischen Lösungsmittel in Gegenwart einer starken Base methyleniert, dann hydrolysiert und das so erhaltene 17α-Hydroxy-1α.2α-methylen-4.6-pregnadien-3.20-dion in an sich bekannter Weise mit einem reaktiven Derivat der Essigsäure in Gegenwart einer starken Mineralsäure acetyliert.

Das erfindungsgemäße Verfahren zur Herstellung von 17α-Acetoxy-1α.2α-methylen-4.6-pregnadien-3.20-dion ist Teil einer Synthese zur Herstellung des bekannten Steroidhormons Cyproteronacetat (6-Chlor-17α-acetoxy-1.2-methylen-4.6-pregnadien-3.20-dion). Nach der bekannten Synthese wird das ebenfalls als Ausgangsmaterial verwendete 17α-Hydroxy-1.4.6-pregnatrien-3.20-dion mit Dimethylsulfoxoniummethylid zur 1α.2α-Methylenverbindung umgesetzt. [DE-B 1.183.500 und Wiechert: "Synthesen antiandrogener Steroide" in Raspé (Herausgeber), Advances in Biosciences, Berlin, 1967].

Dieses Verfahren hat jedoch den Nachteil, daß diese Methylenierungsreaktion nicht einheitlich verläuft, da das Methylenierungsreagenz zum Teil auch an der 20-Ketogruppe angreift. Da die bei diesem Verfahren erzielten Ausbeuten sehr unbefriedigend sind, wird das zur Herstellung von Cyproteronacetat als Ausgangsverbindung verwendete 17α-Acetoxy-1.4.6-pregnatrien-3.20-dion bislang mit Diazomethan methyleniert und die erhaltene Pyrazolinverbindung mittels Säuren gespalten, wie aus der bereits erwähnten Publikation von Wiechert ersichtlich ist. Dieses Verfahren ist aber allein schon aus dem Grund technisch sehr aufwendig und unbefriedigend, weil Diazomethan sehr explosiov und äußerst giftig ist.

Aufgabe der vorliegenden Erfindung war es, ein Verfahren zur Herstellung von 17α-Acetoxy-1α.2α-methylen-4.6-pregnadien-3.20-dion bereitzustellen, das bessere Ausbeuten an Verfahrensproduktes liefert, ohne die Nachteile des Diazomethanverfahrens zu besitzen. Es wurde bereits erwähnt, daß diese Aufgabe gelöst wurde, indem man das 17α-Hydroxy-1.4.6-pregnatrien-3.20-dion vor der Methylenierung mit Dimethylsulfoniummethylid in einem polaren aprotischen Lösungsmittel in Gegenwart einer starken Base an der 20-Ketogruppe mit Ethylenglykol in Gegenwart eines Ameisensäuretrialkylesters und einer starken Säure ketalisiert.

Als starke Säure für die Ketalisierung der 20-Ketogruppe ist insbesondere p-Toluolsulfonsäure oder konzentrierte Schwefelsäure geeignet, die in einer Menge von 0.1 - 2 Moläquivalenten bezogen auf das Ketosteroid eingesetzt werden.

Als polare aprotische Lösungsmittel seien genannt Dimethylsulfoxid, Dimethylformamid, Dimethylacetamid und Hexamethylphosphortriamid. Besonders geeignet ist Dimethylformamid.

Als starke Base zur Einführung der Methylengruppe in 1.2-Stellung ist an sich jede starke Base wie Natrium- oder Kaliumhydroxid oder Natriumhydrid geeignet.

Als starke Mineralsäure zur nachfolgenden Veresterung der 17α-Hydroxygruppe ist an sich jede organische Säure wie Schwefelsäure, Perchlorsäure oder p-Toluolsulfonsäure geeignet, die in einer Menge von 0,05 bis 0,5 Moläquivalenten bezogen auf den Steroidalkohol zugegeben wird.

Der Verlauf der erfindungsgemäßen ersten Reaktionsstufe, d.h. der Ketalisierung der 20-Ketogruppe beim 17α-Hydroxy-1.4.6-pregnatrien-3.20-dion, war an sich nicht zu erwarten, da sich bekanntermaßen 3-Keto-Δ^{1.4}-Systeme unter sauren Bedingungen infolge einer Dienon-Phenol-Umlagerung in Strukturen mit aromatischem A-Ringsystem umlagern (vgl. Djerassi (Herausgeber) "Steroid Reactions" 371-379, San Francisco, 1963 und DE OS 29 13 147). Soweit 20-Ketale beschrieben sind, wird die Δ¹-Doppelbindung eingeführt (J.Med.Pharm.Chem. 5, (1962) 133), die Dienon-Phenol-Umlagerung durch Substitution am

Kohlenstoffatom C-9 und/oder C-11 bzw. C-19 verhindert (DE OS 29 16 889, DE OS 29 13 147, US PS 3 069 417, Helv. Chim. Acta 51, (1968) 1941) oder es werden aufwendige chromatographische Reinigungsschritte angegeben, was auf starke Verunreinigungen durch Nebenprodukte, z.B. durch Aromatisierung, hindeutet (US PS 3 069 417).

Ferner ist bekannt, daß die 20-Ketogruppe schwerer reagiert, wenn sich in Nachbarstellung am Kohlenstoffatom C-21 oder C-17 Substituenten wie z.B. Halogen oder eine Estergruppe befinden (J.Org.Chem. 17, (1952) 1369 und 21, (1956) 65 sowie DE AS 10 00 812, US PS 27 05 720, CA PS 542 851).

Das erfindungsgemäße Verfahren ist somit eine Kombination aus einer neuen erfinderischen Stufe und zwei an sich bekannten Verfahrensstufen. Obwohl das erfindungsgemäße Kombinationsverfahren eine Stufe mehr hat, ist die Ausbeute für das Endprodukt letztlich höher als bei dem bekannten zweistufigen Verfahren, bei dem die 20-Ketogruppe vor der Methylenierung nicht durch Ketalisierung geschützt wird.

Weiterhin hat das erfindungsgemäße Verfahren den Vorteil, daß die Methylenierung statt in Dimethylsulfoxid auch in Dimethylformamid durchgeführt werden kann, das gefahrlos durch Destillation rückgewinnbar und damit umweltfreundlich ist.

Das nachfolgende Beispiel soll das erfindungsgemäße Verfahren erläutern.

### Beispiel

a) 6 g 17α-Hydroxy-1.4.6-pregnatrien-3.20-dion (Trienol) werden in 30 ml Methylenchlorid suspendiert und nach Zugabe von 3,12 ml Ethylenglykol, 4,5 ml Orthoameisensäureethylester und 0,6 g p-Toluolsulfonsäure 3 Stunden bei Raumtemperatur gerührt. Es wird mit 0,6 ml Triethylamin neutralisiert, die Methylenchlorid-Phase mit Wasser gewaschen und eingedampft.
   Ausbeute: 6,9 g Trienol-20-ethylenketal (roh). Nach Umkristallisieren aus Methanol: F. 205 - 207 ^{o}C;
   [α]_{D} = -31,3 ^{o} (Chloroform); UV: ε₂₂₀ = 12.040,
   ε₂₅₃ = 9.340, ε₂₉₈ = 12.600).
   (Diese Verbindung ist bisher nicht beschrieben worden).
b) 6,61 g Trienol-20-ethylenketal (roh) werden mit 6,4 g Trimethylsulfoxoniumjodid und 0,93 g Natriumhydroxid pulverisiert in 66 ml Dimethylformamid suspendiert und 4 Stunden bei 50 ^{o}C gerührt. Die entstandene Lösung wird nach Zugabe von 10 ml 20 %iger Schwefelsäure 2 Stunden bei Raumtemperatur gerührt und dann in Wasser gefällt. Das Kristallisat wird mit Wasser gewaschen und bei 80 ^{o}C getrocknet.
   Ausbeute: 5,42 g 17α-Hydroxy-1.2-methylen-4.6-pregnadien-3.20-dion, F. 232-237 ^{o}C,
   [α]_{D} = +169,3 ^{o} (Chloroform).
c) 5,26 g 17α-Hydroxy-1.2-methylen-4.6-pregnadien-3.20-dion werden in 13 ml Methylenchlorid und 7,3 ml Acetanhydrid gelöst und nach Zugabe von 0,15 ml 70 %iger Perchlorsäure 2 Stunden bei 30 ^{o}C gerührt. Nach Zugabe von 7 ml Wasser wird noch 1 Stunde bei 30 ^{o}C nachgerührt, die Methylenchlorid-Lösung mit Natriumhydrogencarbonat-Lösung gewaschen und das Methylenchlorid nach Zugabe von Methanol abdestilliert. Das Kristallisat wird abgesaugt, mit kaltem Methanol gewaschen und bei 80 ^{o}C getrocknet.
   Ausbeute: 4,46 g 17α-Acetoxy-1.2-methyl-4.6-pregnadien-3.20-dion; Schmelzpunkt 275 - 279 ^{o}C,
   [α]_{D} = +138,6 ^{o} (Chloroform).

## Patentansprüche

1. Verfahren zur Herstellung von 17α-Acetoxy-1α.2α-methylen-4.6-pregnadien-3.20-dion aus 17α-Hydroxy-1.4.6-pregnatrien-3.20-dion, dadurch gekennzeichnet, daß man 17α-Hydroxy-1.4.6-pregnatrien-3.20-dion an der 20-Ketogruppe mit Ethylenglykol in Gegenwart eines Ameisensäuretrialkylesters und einer starken Säure ketalisiert, anschließend das 20-Ketalsteroid in an sich bekannter Weise in 1.2-Stellung mit Trimethylsulfoxoniumjodid in einem polaren aprotischen Lösungsmittel in Gegenwart einer starken Base methyleniert, dann hydrolysiert und das so erhaltene 17α-Hydroxy-1α.2α-methylen-4.6-pregnadien-3.20-dion in an sich bekannter Weise acetyliert mit einem reaktiven Derivat der Essigsäure in Gegenwart einer starken Mineralsäure.

## Claims

1. A process for the preparation of 17α-acetoxy-1α,2α-methylene-4,6-pregnadiene-3,20-dione from 17α-hydroxy-1,4,6-pregnatriene-3,20-dione, which is characterised in that 17α-hydroxy-1,4,6-pregnatriene-3,20-dione is ketalised at the 20-keto group with ethylene glycol in the presence of a formic acid trialkyl ester and a strong acid, then the 20-ketal steroid is methylenated in a manner known per se in the 1,2-position with trimethylsulphoxonium iodide in a polar aprotic solvent in the presence of a strong base, and then hydrolysed, and the 17α-hydroxy-1α,2α-methylene-4,6-pregnadiene-3,20-dione so obtained is acetylated in a manner known per se with a reactive derivative of acetic acid in the presence of a strong mineral acid.

## Revendications

1. Procédé de préparation de la 17α-acétoxy-1α,2α-méthylène-4,6-prégnadiène-3,20-dione à partir de la 17α-hydroxy-1,4,6-prégnatriène-3,20-dione, procédé caractérisé en ce que l'on forme le cétal du groupe en position 20 de la 17α-hydroxy-1,4,6-prégnatriène-3,20-dione avec de l'éthylène-glycol en présence d'un ester trialkylique de l'acide orthoformique et d'un acide fort, puis on forme le méthylène de manière connue en positions 1,2 du 20-cétal-stéroïde avec de l'iodure de triméthylsulfoxonium dans un solvant aprotique polaire en présence d'une base forte, ensuite on hydrolyse et on acétyle de manière en elle-même connue la 17α-hydroxy-1α,2α-méthylène-4,6-prégnadiène-3,20-dione ainsi formée avec un dérivé réactif de l'acide acétique en présence d'un acide minéral fort.
